# EUROPEAN PATENT APPLICATION

(11) **EP 2 522 409 A1**
(43) Date of publication of application: **14.11.2012**
(21) Application number: 11728525.4
(22) Date of filing: 03.01.2011
(51) Int. Cl.: B01D 11/00, B01J 19/10

(54) **DEVICE AND METHOD FOR EXTRACTING ACTIVE PRINCIPLES FROM NATURAL SOURCES, USING A COUNTER-FLOW EXTRACTOR ASSISTED BY A SOUND TRANSDUCTION SYSTEM**

(30) Priority: 04.01.2010 CL 32010
(71) Applicant: Natural Response S.A., Quilpué, Región de Valparaíso (CL)
(72) Inventor: CARES PACHECO, María Graciela, Santiago CP 9170124 (CL); GAETE GARRETON, Luis Francisco Javier, Santiago CP 9170124 (CL); VARGAS HERNÁNDEZ, Yolanda del Pilar, Santiago, CP 9170124 (CL); ALARCON CAMACHO John Gabriel, Con Con (CL); SAINZ LOBO, Javier Ignacio, Con Con (CL)
(74) Representative: Carpintero Lopez, Francisco
(86) International application number: PCT/CL2011/000001
(87) International publication number: WO 2011/079404

(57) **Abstract**

The invention relates to a apparatus and method for extracting active principles from natural sources, using a counter-flow extractor assisted by a sound transduction system, which allows a cavitation sound field to be applied in the zone containing the material formed by the raw material of the natural product and a solvent extraction medium. According to the invention, the apparatus (11) comprises: an inclined casing (12) containing a helical conveyor screw (13) having a plurality of blades (17), said casing including a lower end (15) and an upper end (16); a material inlet (21) for introducing the material, which is disposed on top of the lower end (15) such that it is inclined at an angle *θ* in relation to the surface of the casing (12), said material inlet (21) including a second helical conveyor screw (22); an discharge hopper (31) for releasing the treated material, which hopper is located at the upper end (16) of the casing (12); a first load line (23) for loading the solvent extraction medium, which load line is located at the upper end (16); a discharge outlet (26) for discharging the liquid extract, which discharge line is located at the lower end (15) and is provided with a filter (27); and a sound transduction system (29) for producing ultrasound, which is located at the lower end (15) on a surface portion (28). The method comprises the following steps: (a) preparing the raw material; (b) supplying the product and the solvent to the device, in counter-flow mode; (c) applying a sound field to the product together with the solvent; (d) extracting the product with the extraction liquid, in counter-flow mode; and (e) collecting the liquid extract and the depleted material.

## Description

### OVERVIEW OF THE INVENTION

The present invention comprises an apparatus and method for extracting active principles from natural sources. The key to the present invention is the optimal combination of a counter-flow conveyor screw extracting system with the application of a high-intensity sonic field. The invention refers to a continuous extraction in a device where through one of its ends is fed the source material while a fluid is loaded in counter-flow by the opposite end, allowing the variation of extraction parameters along the entire length of the continuous extractor, such as, for example, temperature, the liquid/solid ratio and the residence time. The use of ultrasound improves extraction performance at a given temperature, allowing for low liquid/solid ratios and enhancing the extractor's performance compared to a normal continuous extractor.

The present invention has significantly greater comparative advantages than state-of-the-art apparatuses and methods: it has lower power consumption; uses fewer solvents; operates at lower temperatures, reducing thermal energy requirements; it has the advantage of low liquid/solids ratio; it minimizes the area and cross-section necessary for the application of sonic energy; it requires less applied acoustic power, reducing the size of the investment required for its installation and operating expenses, among other advantages.

### BACKGROUND INFORMATION

During the extraction of active principles from natural sources, it is necessary many times to extract at low temperatures, low liquid/solid ratios and short processing times. The idea is to avoid the degradation of components and excessive subsequent concentrations (relative). However, these simple restrictions mentioned above cause devastating effects on the yield of the processes currently in use. Normally are frequently used sub-optimal solutions of process that favour yield over quality, even without achieving optimal yield and causing the components degradation due to the use of high thermal energy.

At the moment, the obtention of active principles from natural sources is made by using batch-type techniques or continuous extractions. The extraction alternatives currently existing on the market and references for the present invention are the hollowing:
1. Batch-type and counter-flow extraction systems.
2. Continuous extraction systems.
3. Ultrasonic extraction.

The systems most used in the industry are the batch-type extraction systems, where these processes have multiple applications related to extraction stages in recirculation tanks that can be adapted to simulate a semi-continuous counter-flow process. However, batch extractors do not take advantage of the mass gradients between components; they use high liquid/solid ratios and multiple rinses, using large amounts of solvents per unit of solids extracted.

Currently, continuous extraction systems work mainly in the counter-flow mode, i.e. the input component (solute, raw material etc.) meets the collecting solvent in the opposite direction within the extractor. Thus, each portion of volume of the substance to be extracted is always surrounded by a volume of unsaturated solvent, thus avoiding the suspension of the process due to imbalance.

Continuous conveyor screw extracting systems have multiple applications and there are a wide range of offers of equipment for companies worldwide. These types of processes are commonly used in the food industry to separate soluble fractions of several types of raw materials, since they offer lower solvents use and are capable of recovering the extract in high concentrations. There are a considerable number of patents describing these types of counter-flow extractors and/or their utilization methods. One of the closest document to the present application is the patent US 5,409,541: *"Method and apparatus for extracting soluble and dispersible materials from products using a slotted scroll extractor"* from to David R. Walter. The Walter's patent describes a method and apparatus for the extraction of soluble and insoluble solids from products by means of a conveyor screw extractor. More precisely, Walter's extracting apparatus comprises a conveyor screw contained in a casing formed by a hollow stainless steel semi-cylinder and equipped with semi-circular walls at its ends to contain the material being treated. The casing is surrounded by a system, forming a double wall along the extraction tank, allows to control its internal temperature. Furthermore, Walter's apparatus for extracting has the capacity of inputting different solvents in different sections of the conveyor screw, achieving a process that is characterized in that, in a first stage, the solvent is water, and in the second stage is alcohol. That is to say, the invention disclosed by patent US 5,409,541 describes a simple extraction method with an apparatus to realize it, that allows for multi-stage extraction in which are used different means of extraction and/or different solvent input flows in the different sections of the extractor.

Counter-flow extractor devices have had positive results; nonetheless, the process does not achieve optimal standards. The input material tends to gather in the casing of the extracting apparatus, forming a compact layer, whereas the solvent tends to flow along the surface of the casing without efficiently engaging the raw material, leaving inside the extractor apparatus a significant part of the desired active principles. Another one of the most significant disadvantages of counter-flow extraction devices is the high solvent content necessary for improving the process, as the *diffusion* only reaches optimal levels in those parts of the conveyor screw in which the input material is partially submerged. To solve these problems are used suboptimal process solutions, such as for example, the possibility of re-loading the source material (or the extract) into the equipment in order to improve the process efficacy. However, there are some natural sources that are affected due to the growth of toxin-producing microorganisms, which are not allowed in some applications. In order to eliminate the toxins generated in the final extract, finer and technically more complicated processes must be used, which is usually costly procedures, since they recur to high temperatures and the use of semipermeable membranes.

There are other continuous extraction systems with the application of acoustic microwaves. The procedures for extracting natural products using acoustic microwaves disclosed in the prior art, can be made to work onto small masses of biological material, due to the power of the applied microwaves. These methods generally apply power ranges between 10 and 20 kW per kilogram of biological material, using water as a solvent. Therefore, these procedures cannot be executed at an industrial scale, since use of the technique becomes prohibitive due to the investment and power requirements.

The patent WO 1994026853 *"Method and plant for solvent-free microwave extraction of natural products"* from Philippe Mengal and Bernard Mompon, discloses a method and apparatus for extracting natural products from biological material. The invention of Mengal *et al* consists in submitting the biological material in the absence of solvents to acoustic microwave radiation (2450MHz). This enables the hydrodistillation of the material that it is wanted to be extracted, not by the water provided from outside during the process, but rather by the water contained in the treated biological material (about 30% in biological materials). The invention described by Mengal *et al* has comparative advantages with respect to the traditional methods of extraction using microwaves: one obtains a sample virtually free of all solvent residues, and high yields compared to the traditional hydrodistillation process.

Apart from its low application cost, this technique has the advantage of providing the acquisition of free residual solvents extracted products, thus obviating the need for subsequent residue-elimination treatments. It is worth mentioning that there are several products that cannot be extracted from the natural material that contain them using these methods, and for which it is necessary to use extraction techniques that use organic solvents.

The use of ultrasound for extracting the main bio-principles of plants has been considered in a wide range of studies; there are studies on the application of ultrasound for extraction that date back to the 50s of the 20th century, a decade where it was intensively explored the use of ultrasound in different processes. In contemporary studies, like the one by Mircea Vinatoru, described in the article *"*An overview of the ultrasonically assisted extraction of bioactive principles from herbs", Ultrasonics Sonochemestry, 2001, vol. 8, 303-313, there is a complete comparison of the different extraction methods developed to date applied to vegetable material (distillation, extraction with solvents, cold compression and unconventional techniques), highlighting the capabilities of ultrasound when gauging the efficiency and the efficacy of processes. Furthermore, Vinatoru's study describes the first extraction tank for the extraction of vegetable products with ultrasound assistance. The research was carried out through the EU COPERNICUS (ERB-CIPA-CT94-0227-1995) Program. The extracting apparatus has a capacity of 1 m³, has four transducers radiating from the walls of the extraction tank. The technique described by Vinatoru provides 50% improvement in process efficiency compared to the traditional process.

Currently, it is accepted that ultrasounds can stimulate different extracting processes. In the work of Kamaljit Vilkhu, especially his article *"*Applications and opportunities for ultrasound assisted extraction in the food industry, a review", Elsevier, 2008, vol 9 161-169, it is suggested that the implosion of micro-bubbles generates macro-turbulence, collisions between particles and excitation of the micropores of the biomass particles, which accelerates internal and "Eddy" diffusion. The improvement in the efficiency of the ultrasound-assisted extraction process is attributed to the propagation of high intensity sonic pressure waves in fluids, particularly due to the phenomenon of *sonic cavitation.* It is worth mentioning that in order to increase the efficiency of the ultrasound-assisted process, the size of the particles of the extraction material must be reduced, since there is a relationship between their size and their interaction with the sonic field existing in the extraction tank, as shown in the article by Balachandran, Kentish, Mason and Ashokkumar (2006), "Ultrasonic enhancement of the supercritical extraction from ginger", Ultrasonics Sonochemistry, vol. 13, 471-479. When reducing the size of the chips of the material to be treated, it increases the contact surface directly exposed to the ultrasonic radiation, thus favouring the transfer of the mass of the soluble components from the input material to the solvent.

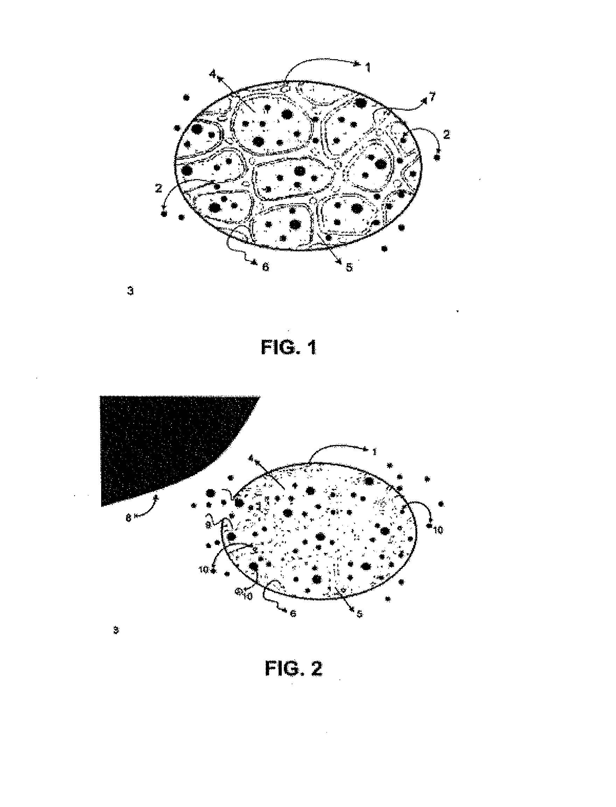

**FIG. 1** shows a schematic sketch of a vegetable cellular structure **1** in which the material particles **2** migrate to a solvent medium **3.** The vegetable cells **4** are separated from the intercellular medium **5** and from the other cells by their cellular wall **6.** The vegetable cells are delimitated by their cellular wall **6** and their plasmatic membrane **7.** The plasmatic membrane **7** is a structure which encloses the cell, defines its limits and contributes to maintaining the balance between its interior and exterior, whereas the cellular wall **6** is the layer that covers the cellular membrane, preventing changes of form and position, its function is to protect the cell. When applying ultrasound to the extraction process, the contact between the solvent and the solid is improved due to the phenomenon of cavitation of the ultrasound waves. These cavitation micro bubbles open spaces (capillaries) and favour the entry of the solvent, improving hydration. They also enable the extraction of compounds sensitive to heat due to the reduction of thermal energy, and offer shorter process duration. In **FIG. 2** it is possible to observe the same vegetable cellular structure bombarded by ultrasonic pressure waves **8;** these are the waves that generate the acoustic cavitation bubbles. As the cavitation bubbles implode close to the solid/liquid interface, the jet of fluid produced by the implosion impacts the surface of the cell, generating macro-turbulence, collisions between particles and excitation of the micropores of the biomass particles, damaging the cellular wall **6** and subsequently rupturing it **9,** thus improving diffusion **10** and thereof improving the extraction process.

The patent WO 2005087338 *"Process for extraction of diterpenes and triterpenes from biomaterial',* from Stevanovic and Lavoie, discloses an ultrasonic process for the extraction of diterpenes and triterpenes from biological material. Stevanovic's patent includes a comparison between the potential of two traditional extraction methods *(Soxhlet* and Maceration) and ultrasonic extraction. The extraction techniques developed use organic solvents such as methanol, dichloromethane, hexane and ethyl acetate.

The *Soxhlet* type extraction method causes modifications in certain types of thermally unstable molecules when applying high temperatures. On the other hand, the maceration techniques studied do not use high temperatures, but show less efficiency of the process. In all the experimentation cases studied, ultrasonic extraction shows an increase in the efficiency of the process, achieving a reduction in extraction time from six days to thirty minutes. Nonetheless, it is worth mentioning that the ultrasonic extraction process described in the document WO 1994026853 has been laboratory tested for reduced volumes of solution with a 12.7mm (0.5inches) radiant panel of the transducer. The high volume implementation of this technique, due to the high intensity of the sonar waves, necessary for obtaining efficient energy transmission to high volumes of product, would make prohibitive its use on an industrial scale.

There are initiatives in which ultrasound has been applied on an industrial scale, where extraction alternatives have been developed by batches with agitation systems. The industrial application of these processes basically depends on the technology used for generating the high intensity ultrasound. The main aspects to consider in the ultrasonic transducers are their power capacity, yield, magnitude, distribution of the vibration and the directionality of the radiation emanated. Financially it is profitable the introduction of an *ad-hoc* device in the industrial process and to do this it is requited to develop of specific sonic radiators, since in this case probably the sonic field is more directional, magnitudes are greater and more numerous pure field effects can be expected than in the case of the ultrasonic baths whose radiation is substantially diffused.

Even though the ultrasound-assisted extraction devices have given positive results, they have disadvantages such as: the active region of the *sonication* process is restricted to the surroundings of the radiant panel of the transducer; the sonic radiation area must have a high liquid/solid ratio, which reduces the economy of the extraction process due to the need of concentrating the resulting extract; the generation of sound energy in fluids is problematic due to the low acoustic impedance (pC) and the high absorption of these means. Hence, in order to obtain efficient energy transmission and produce high intensities, it would be necessary to achieve good acoustic impedance adaptation between the transducer and the fluids, large vibration magnitudes of the transducer and high-energy concentration. Due to the high intensities required for obtaining efficient energy transmission in the process, it is difficult to coordinate that product volumes are increased and the processes continuous.

The patent EP0243220 *"Procédé et dispositif detraction par ultrasons de produits oléagineaux à partir de graines aléagineuses"* from Bernhard René Guillot, discloses an ultrasound-assisted extraction process. The process comprises several extraction subprocesses: (1) Mechanical treatment of the material (grinding, crushing), (2) Percolation or extraction by solvents (batch processing with agitation), (3) Ultrasonic extraction, (4) Rinsing Stages (at least one) and, finally, (5) *micelle* treatment for recovering the solvent. The described method uses ultrasound for breaking down the links between the unextractable components of the residual oil, by using traditional methods (percolation, solvents). The extraction process described in Guillot's patent achieves practically total extraction of the oleaginous material (99%). Nonetheless, it is worth mentioning that this technique does not take maximum advantage of the mass gradients between the studied components. In the process, are performed multiple extraction and rinsing stages, using a large amount of solvents per unit of extracted solid.

This aforementioned prior art information, reveal that the ultrasonic and counter-flow extraction methods and devices for obtaining active principles from natural sources are efficient under specific conditions; each method has advantages and restrictions when employed on an industrial scale, either due to high energy costs, the amount of raw material to be treated, the types and quantities of solvents, high temperatures in the processes, lengthy extraction times etc.

Continuous extraction using conveyor screws stand out for achieving high yield with low liquid/solid ratios; ultrasonic extraction improve extraction yields and enable obtaining higher yields at lower temperatures. In general, the use of continuous conveyor screw extractors allows for industrial scale efficiency, although it still does not offer improvements with regard to the use of batch type extractor options. This problem can be resolved by incorporating ultrasonic means. However, the low liquid/solid ratios in continuous extraction system are not favourable for the application of an ultrasonic field. The conditions under which it would be profitable to apply ultrasound are not offered by the currently available counter-flow extraction systems.

The above shows that it is convenient to introduce some nexus that would allow the inclusion of both extraction techniques for achieving greater efficiency in the processes.

The extraction system developed in the present invention efficiently integrates the advantages of a continuous system (low overall liquid/solid ratio) with the advantages of a batch system (specific areas of high liquid/solid ratios). In other words, advantage is taken of the fact that while the overall liquid/solid ratio is low (5:1), there are flooded areas where the liquid/solid ratio reaches values close to 15:1. This makes it feasible to apply an acoustic cavitation field in the flooded area, thus reducing the disadvantages of extensive areas and volumes for the propagation of the ultrasonic wave, factors which make the use of this technique prohibitive due to the investment requirements.

This new operating mode, not described in the prior art, constitutes one of the most relevant aspects of the present invention: a coupled transducer system capable of producing a highly cavitational environment, that generates a high density of liquid micro-currents and the presence of shock waves that produce local depressions that facilitate the extraction of the active principles of the matrix during the counter-flow extraction stage. When merging both extraction techniques it is possible to take advantage of the system liquid accumulation areas for efficiently applying the ultrasonic field in a reduced section of the extracting apparatus. The above optimizes extraction both in terms of power and investment effectiveness, giving rise to a feasible alternative for the industrial adoption of the technology.

The potential of the present invention reduces the thermal energy requirements, maintains the overall liquid/solid ratio; minimizes the acoustic energy propagation area and the acoustic power of the system, thus reducing the magnitude of the investment required in the installation and operating stages. The combined use of both extraction techniques also enables obtaining lesser amounts of undesired components, enhancing the purity of the active component in the extracted solids.

The field of application of the present invention is very broad, since it covers several industries that perform extraction in the agro industry area.

### DESCRIPTION OF THE DRAWINGS

The attached drawings are included for providing a better understanding of the invention. They are part of the present invention and together with the detailed description, serve to explain its principles.
**FIG. 1** is a diagram of a cellular structure of a natural vegetable product of the prior art treated with a counter-flow extraction method in which the extraction medium is water;
**FIG. 2** shows a diagram of a cellular vegetable structure of a product of the prior art treated with ultrasound, in which the extraction medium is water;
FIG. **3** shows a profile cross-section view of the extracting apparatus, without showing the driving parts, for the purpose of greater clarity;
FIG. **4a** shows a profile view of the lower part of the extracting apparatus in **FIG:3****,** without showing the driving parts;
**FIG. 4b** shows an elevation of the lower part of the extracting apparatus in **FIG. 3****,** without showing the driving parts, illustrating the ability to radiate from the sides of the extractor;
**FIG. 5** shows a schematic block diagram of the excitation system and the acoustic adaptation used for applying the ultrasound assistance to the process;
**FIG. 6** shows a chart with the results of a preliminary study carried out for determining the influence of ultrasound on the extraction process of Quillay saponins and extractable solids, comparing the traditional method, which entails 3 hours of extraction at 60°C, with the ultrasonic method of the invention, which requires 20 minutes of extraction at room temperature. The chart also shows the results of the extraction at room temperature (20° C), without ultrasound;
**FIG. 7** shows a chart with the results of a study carried out for determining the influence of ultrasound on the extraction process of Quillay saponins, using the concentration of the sample as a variable (95% reliable results). The samples were taken at room temperature;
**FIG. 8** shows a chart with the results of a study carried out for determining the influence of ultrasound on the purity of Quillay extraction and the relationship between purity and particle size (95% reliable results). The samples were taken at room temperature; and
**FIG. 9** shows a chart with the results of a study carried for determining the influence of ultrasound on saponin extract using the HPLC method (High Performance Liquid Chromatocharty).

### DETAILED DESCRIPTION OF THE INVENTION

The present invention offers a method and an apparatus for extracting active principles from natural sources, combining counter-flow extraction with the application of a high-intensity sonic field.

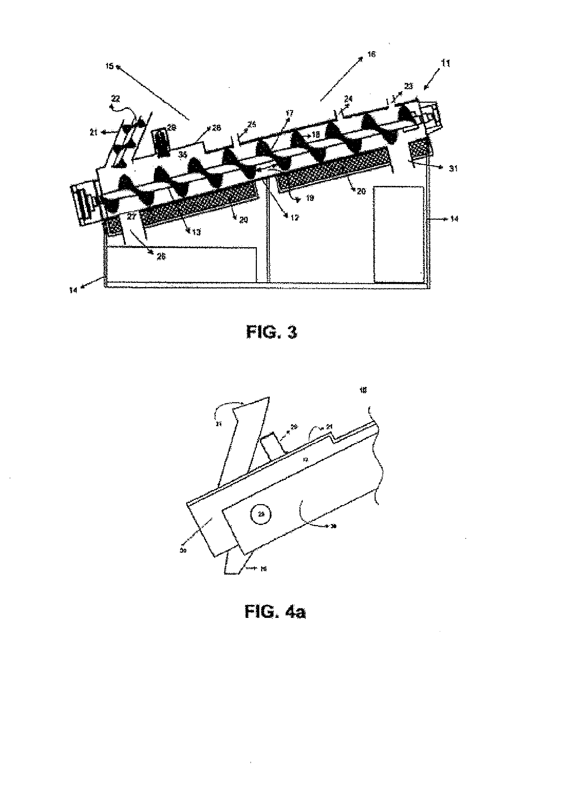

The extracting apparatus **11,** the subject matter of the present invention, is shown if **FIG.** 3. The extracting apparatus **11** comprises a continuous counter-flow extractor, assisted by ultrasonic means. The conveyor screw units currently commercially available can be modified and made to function with good results, incorporating into the extracting apparatus **11** an ultrasonic transducer system, with the modifications of the present invention described below.

The extracting apparatus **11** has a casing **12,** with the shape of a channelled or cylindrical sump (truncated cone shape), which encloses and supports an initial conveyor screw **13.** The casing **12** of the extracting apparatus **11** can be any structure capable of containing the initial conveyor screw **13** and the casing can be inclined at different angles thanks to a support structure **14** fitted with means of inclination that can be regulated. The angle of inclination of the initial conveyor screw **13** will depend mainly on the characteristics of the raw material to be extracted, falling within a range of 5° to 85°, the idea being that the rest of the extracting apparatus **11** will be partially submerged with the solvent. Thus, any volume portion of the material to be extracted will always be covered by an amount of unsaturated solvent, avoiding that the extraction process is locally suspended. In a preferred embodiment of the present invention, the angle of inclination of the initial conveyor screw **13** is 15°.

The material to be processed that enters the extracting apparatus **11** is raw material comprising any kind of vegetable source from which some kind of product can be extracted, such as for example: coffee, tea, tobacco, almonds, or in general, fruit, wood, plants or parts of them, or else a combination of the above.

In order to transport the material through the conveyor screw, an engine makes the initial conveyor screw turns **13.** The engine can be hydraulic, electric, or any other device capable of imparting unidirectional rotational movement to the initial conveyor screw **13,** in such a way that it will enable transferring the raw material in counter-flow with a fluid from the extreme low end **15** to the extreme high end **16** of the extracting apparatus **11.** The initial conveyor screw **13** includes a plurality of pallets **17** which jointly make up the helix of the conveyor screw **13.** Each pallet **17** has grooves **18** for increasing material-solvent contact. In the preferred embodiment of the present invention, the grooves **18** have a radial configuration, perpendicularly extended to the axis of rotation of the conveyor screw **13.** In this way the probability of the product getting stuck in the grooves **18** is reduced. The distance between pallets **17** depends on the properties of the material to be extracted.

Temperature regulators means **20,** are located around the casing **12** of the extracting apparatus **11** and are designed for indirectly heating or cooling its inside. The means of transferring thermal energy of the temperature regulators **20** can be steam, hot water or any other suitable fluid.

The extracting apparatus **11** has at least one material inlet **21** which is located on the upper side of the lowest end **15,** inclined at an angle θ to its surface. The material to be introduced through said material inlet **21** is previously fragmented. This material inlet **21** enables the continuous input of raw material through a second conveyor screw **22,** similar to the initial conveyor screw **13** of the extracting apparatus **11.** Once the raw material has been extracted, it is discharged into at least one material discharge hopper **31,** located on the extreme upper end **16** of the extracting apparatus **11.**

On the upper end **16** of the extracting apparatus **11** there is at least one initial liquid loading line **23** for injecting the fresh extraction solvent. In order to improve the diffusion of the extraction process, additional fresh extraction solvent loading lines can be located in other positions on the extracting apparatus **11,** such as, for example, a second liquid loading line **24** and/or a third liquid loading line **25,** located at other points on the surface of the casing **12,** between the initial liquid loading line **23** and the portion of the lower superficial layer **28** of the extracting apparatus **11.**

At the lower bottom end **15** of the extracting apparatus **11** there is at least one liquid extract discharge outlet **26** fitted with an exit sieve or filter **27,** which filters the final extract prior to evacuation.

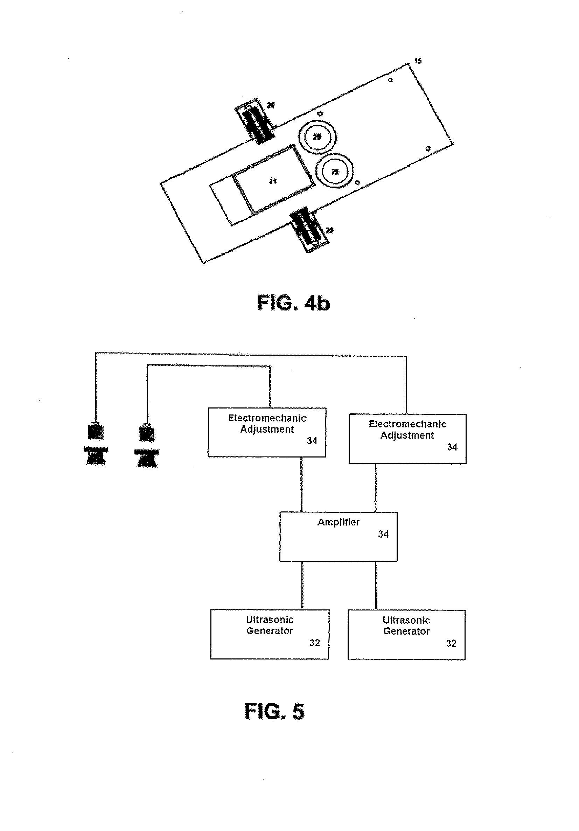

At the lower bottom end **15** of the extracting apparatus **11** there is a superficial portion **28** of the casing **12,** upon which the sonic transducer means are fitted **29,** which are benefited with the flooding with extraction solvent of a small portion of the extracting apparatus **11,** favouring counter-flow extraction. This design enables all the particles of fragmented material that have been previously reduced, confront the sounding field evenly. **FIG. 4a** and **FIG. 4b** illustrate the radiating capacity from the sides **30** of the casing **12** of the extracting apparatus **11** to increase the intensity of the sonic radiation.

The sonic transduction means **29** are high-intensity sonic transducers. These sonic transducers must radiate the surface of the raw material directly in the extract in order to avoid the raw material screens the sonic radiation, which would be detrimental for cavitation and therefore would be detrimental to the extraction process. The superficial portion **28** which supports the sonic transduction means **29** is at a different level than the structure of the extracting apparatus **11** and between this structure and the casing **12** of the extracting apparatus **11,** there is a mesh **35** which holds back the raw material.

One of the embodiments in which the present invention can be realized is illustrated in **FIG.** 5, which shows excitation devices for a piezoelectric transducer. The excitation devices in this embodiment of the invention consist of the corresponding self-engaging ultrasonic generators **32** for the respective transducers; signal amplifying means **33** and an electromechanical adaptation network **34.** The modern ultrasound systems include automatic frequency scanning systems for ensuring that the maximum amount of energy will be transmitted to the system.

The sonic transduction means are fitted with a sonic wave generating device and means for adapting the sonic impedance in order to facilitate the emission/reception of the sonic waves used (electromechanical adaptation). These sonic transducers can have a radiant area in the shape of a disc, stepped horn, plate, among others. The sonic transducers used in the preferred embodiment of the invention will preferably have a resonance frequency between **15** kHz and 45kHz. These sonic transducers can be located in any section of the extracting apparatus **11,** as long as it favours sonic cavitation in the extract.

The method used in the present invention is continuous, counter-flow extraction, assisted by sonic means. The natural source to be extracted must be of vegetable, fruits or other organic nature, such as for example: coffee, tea, tobacco, almonds, fruit, wood, plants or parts of them.

The method consists in several stages which are listed below:

### 1. Preparation of the Raw Material

In order to increase the efficiency of the process, one must first grind or reduce the size of the source material to between 6 and 80mm. By reducing the size of the material, the material-solvent contact surface increases, favouring the transfer of the mass of the soluble components from the material to the solvent. Subsequently, and depending on the properties of the biological material to be extracted, the process may or may not include a reduction stage in which the degree of humidity of the material, either by means of heat, pressure, vacuum drying, or any other method that does not alter the properties of the material.

### 2. Fine-Tuning of the Apparatus

In the present invention, extraction occurs in a continuous extractor, like the one illustrated in **FIG. 3****.** As previously mentioned, the continuous extractor is fitted with ultrasonic capabilities. The preferred and typical parameters for the optimal functioning of the extracting apparatus **11** for the extraction of active principles must be established by means of batch-type trials. Each factor must be assessed in accordance with industrial process parameters.

The most important factors in sonic extraction are: particle size of the product, exposure time to the sonic filed, acoustic power, concentration (liquid/solid) and temperature. In general, each one of the variables will depend on the solvent-material-sonic field interaction, which must be considered in detail. It is important that the material should be exposed to the sonic field as evenly as possible. In this fine-tuning stage of the extracting apparatus, sonic cavitation acts on the cellular wall of the material, optimizing the next extraction stage, improving mass transfer.

For the counter-flow extraction process, the additional factors that must be considered when planning the continuous extraction process are; the backmixing effect, the raw material feeding rate, the angle of inclination of the conveyor screw, liquid/solid ratios, insoluble solids, the solvent feeding rate, the product feeding rate, the speed of rotation of the conveyor screw and temperature.

In general, in order to establish the values of the efficiency parameters in the counter-flow extraction process, it will be necessary to study each one of the factors mentioned, as well as their possible interactions.

### 3. Extraction Stage

The extraction stage is performed in counter-flow in an extracting apparatus **11,** like the one described, in which fluid is injected through one of its ends and the input material enters through the other end. The raw material enters the extracting apparatus **11 (****FIG. 3****)** from a material inlet **21** located at its lower end **15** to make contact in counter-flow with the extraction liquid, which is injected through the upper part of the initial liquid loading line **23** of the extracting apparatus **11.** The extraction liquid ― or solvent ― used will preferably be water, but it can be any kind of solvent compatible with the requirements of the process and the substance to be extracted, such as for example: alcohol, chloroform, methanol, acetone or hydroalcoholic mixtures.

The extreme lower end **15** of the casing **12** of the extracting apparatus **11** is flooded to the superficial portion **28,** which contains the ultrasonic transduction means, whose radiant area must be submerged. The solution formed between the raw material and the extract, creates the conditions for the application of the sonic field. The sonic field affects the cellular wall, increasing the permeability of the tissues of the material, improving the transfer of mass and optimizing counter-flow diffusion. The exposure time of the raw material in the sonic field and its intensity will depend mainly on the properties of the material-liquid mix being used. Once the source material has been subjected to the sonic field, it will continue to be raised counter-flow together with the extraction liquid, so that the delivering component (product, solute, raw material etc.) is moving in the opposite direction to the gathering solvent. The rest of the extracting apparatus **11** must remain partially submerged, depending on the angle of inclination (70% of the extracting apparatus **11).** Thus, any portion of the volume of material to be extracted always comes into contact with a volume of unsaturated solvent, avoiding the local suspension of the extraction process. During the process, the temperature of the extracting apparatus **11** can fall within a range of 16°C and 80°C, by means of the temperature regulation means **20** and of the solvent, with the preferable temperature being 40°C. Once the counter-flow extraction process has concluded, the extract rich in active principles goes through the discharge outlet **26** of the extracting apparatus **11,** through the filter **27** installed at the end of the extracting apparatus **11** and is collected to go through different external purification processes. The exhausted residual material is discharged from the extracting apparatus **11** through at least one discharge hopper **31,** located on the upper end of the extracting apparatus **11.**

### APPLICATION EXAMPLE

### Extraction of Active Principles of Quillaja Saponaria Molina.

### Batch-type ultrasound experiments

The Quillay is an indigenous tree of Chile *(Quillaja Saponaria Molina),* whose bark contains significant amounts of saponins. The saponins present in the Quillay are alkaloids of the triterpenoid type produced during the secondary metabolism of these trees. Saponins have a triterpenic nucleus, with two sugar chains linked to it. The sugar chains endow the saponins with a hydrophilic nature, whereas the triterpenic nucleus confers a hydrophobic nature to them, converting them into amphoteric molecules. The main effects of these molecules are: reduction of surface tension, formation of persistent foam, emulsion of fats and oils, reduction of ammonium, activation of microbial growth etc.

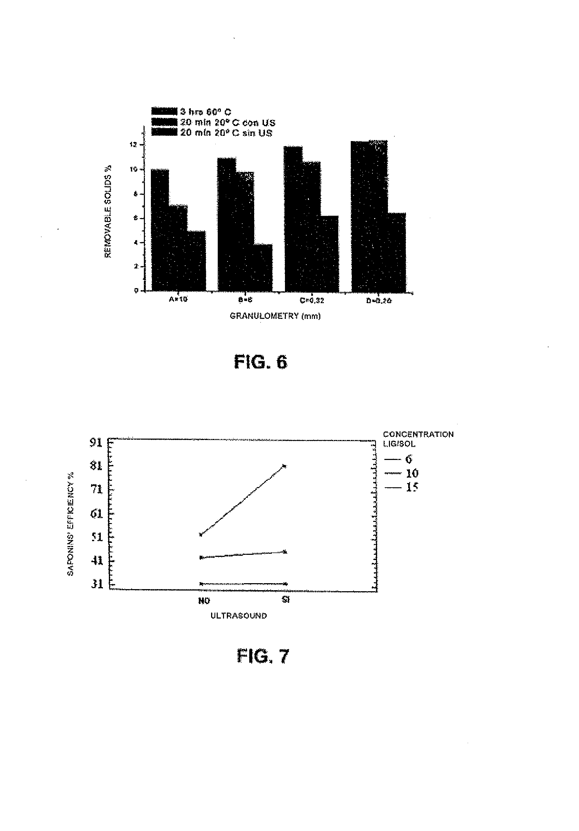

In **FIG. 6** we can see the advantages offered by ultrasound compared to the traditional method in the extraction of active principles of Quillay in batch-type samples, applying extraction according to the present invention. The results show that the chips of the material to be processed, when submitted according the invention to a sonic field for 20 minutes at 20°C, achieve total extraction in dust-type particle sizes, which demonstrates the power of the sonic system of the invention compared to the traditional process, which requires 3 hours at 60°C to achieve similar extraction. This at least shows that there is a clear and significant influence of the ultrasound used in the Quillay saponins extraction process.

In order to establish the sonic parameters for the extraction of the active principles of the *Quillaja Saponaria Molina* (Quillay) the factors that influence the process were studied. To do so, the extraction process in the plant was simulated using an experimental design. The experimental design was performed with coherent levels that produce effects that can be reproduced on an industrial scale. The transduction system used for the batch type samples was a vibrator comprising a piezoelectric transducer of the Langevin type, coupled to a stepped mechanical amplifier with a resonance frequency of 20kHz. The influence of each effect and its interactions were studied through experimental factorial designs, which enabled finding statistical significance in the answers. Each factor must be assessed on the basis of coherent levels with the industrial process. The factors studied are *Concentration, Acoustic Power, Exposure Time to the Field and Particle Size of the Chips.*

**FIG. 7** illustrates the influence of a cavitational sonic field, particularly ultrasound, in the experimental process carried out on samples of the batch type, according to the concentration of the samples. These results show the feasibility of using the specific 15:1 high liquid/solid ratio areas to advantage for the sonic treatment of the chips of the material to be treated, with the acoustic cavitation phenomenon of ultrasonic radiation being indispensable in the extraction process.

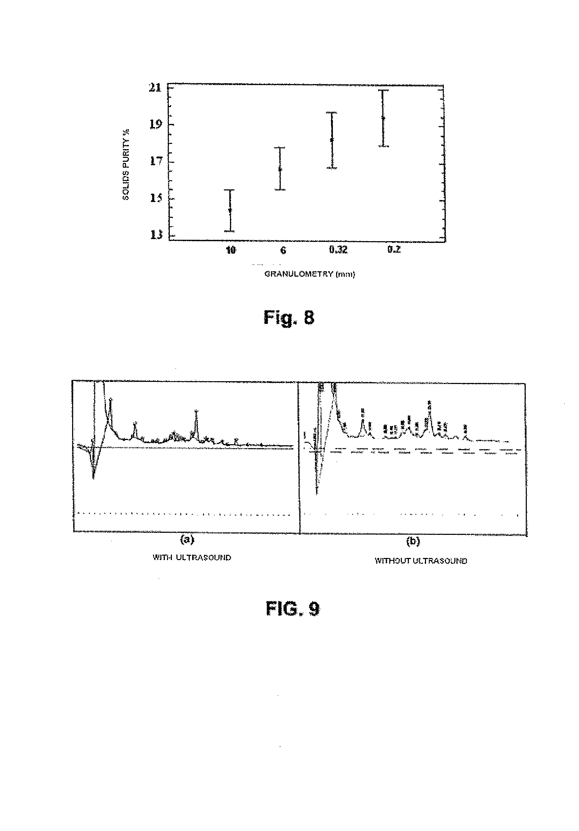

Another one of the significant advantages of the sonic method of the present invention lies in its simple selectivity capability, since it achieves a reduction in the extraction of unwanted components. This is illustrated in **FIG. 8****,** in which the percentage of purity of the extracted solids increases when reducing the size of the chips.

Another important aspect to consider in the ultrasonic extraction process when using the present invention is the quality of saponinic profiles. **FIG. 9** illustrates the saponinic profiles of the samples analyzed with HPLC in which: **(a)** is the sample submitted to a sonic field, whereas **(b)** is the profile of the sample in standard conditions, without the sonic field.

The use of sonic radiation in the extraction of Quillay saponins, applying the present invention, has improved the process, optimizing it in terms of power, effectiveness and investment, producing a feasible alternative for the industrial establishment of the technology. On one hand, the time required for the total extraction of saponins from the wood has been reduced compared to the traditional method in batch-type samples **(****FIG. 6****)** and, on the other hand, the extraction temperature has been reduced, which optimizes selectivity **(****FIG. 8****),** yield and energy consumption.

### Ultrasound-Assisted Continuous Counter-flow Extraction Procedure

First of all, the wood has to be cut into pieces (in this case, the bark) in order to reduce the size of the particles of raw material. The raw material must preferably be reduced to fibrous chips, with average diameters within the range of 6 to 80 mm. The product is then sieved to remove dust and avert clogging in the output filter **28** of the extracting apparatus **11.**

Extraction usually starts by adding 3kg of bark through the material inlet **21** of the extracting apparatus **11,** while the solvent (distilled water in this case) is loaded through the fluid loading line **23** in counter-flow to the raw material. The solvent input flow must be increased until the radiant faces of the ultrasonic transduction means **29** are fully submerged. The flooding level must be kept constant so as not to vary the acoustic impedance of the system (extractor-transducers-raw material-solvent), taking into account the option of loading additional solvent at different flow rates through the different fluid loading lines **24** and/or **25** of the extracting apparatus **11** so as to enhance solvent-material contact. The water temperature should preferably be at 40°C, in order to prevent the proliferation of microorganisms in the final extract. It is important to work at high concentrations of liquids/insoluble solids, since the wood is capable of absorbing a large amount of liquid, increasing its size and reducing the possibility of all the bark rising through the conveyor screw **13** coming into contact with the solvent, thus impeding the mass transfer effect.

With the raw material and the ultrasound means arranged as described, the ultrasonic field is then applied. The solvent loading input and extract output flows must be kept constant in order to avoid modifying the filling volume in the extracting apparatus **11.** As the raw material moves through the conveyor screw, it comes into contact with progressively purer solvent, thus facilitating extraction. The final extract, rich in active principles, passes through the output filter **27** installed at the end of the extractor and is collected for subsequent pasteurization. The exhausted bark is discharged through the discharge hopper **31,** located at the upper end of the extracting apparatus **11.**

The extracting apparatus **11,** fitted with sonic devices capable of generating cavitation, enables high yield extraction at low temperatures, taking advantage of the liquid accumulation areas in the system for efficiently applying the sonic fields in a reduced section of the apparatus, optimizing its application in terms of power, effectiveness and investment cost, providing a feasible alternative for the industrial scalability of the technology ― all the above without increasing the overall liquid/solid ratio, minimizing the area and cross section of acoustic energy application, the power output, and consequently, the size of the investment required.

## Claims

1. An apparatus for the extraction of active principles from natural sources using an extractor on counter-flow assisted by an acoustic transducer system, which allows the application of an cavitative acoustic field in the area flooded with the material comprised of the raw material of the natural product and a solvent extraction means, where such apparatus **(11)** is **CHARACTERIZED in that** it comprises:
a case (12) that surround and supports a first helical Conveyor screw (13) with a plurality of vanes (17) which form such helicoids, being such case bended in an angle between 5° and 85°, having the case (12) a bottom end (15) and a top end (18);
at least an inlet hopper (21) of the material is available over the bottom end (15) of the apparatus (11) in a bended way respect to the top surface of such apparatus (11), having the inlet hopper (21) a second helical conveyor screw (22);
at least an outlet hopper (31) to unload the processed material, which is located at the top end (16) of the case (12);
at least one first load line (23) to load such solvent extraction means located at the top end (16) of the case (12);
at least one unload line of the liquid extract (26), located at the bottom end (15), being that unload line (26) provided with a sieve (27) that filtrates the liquid extract; and
an acoustic transducer system (29) to produce cavitation located at the bottom end (15) over a superficial portion (28) of the case (12).

2. An apparatus for the extraction of active principles, according to claim 1, **CHARACTERIZED in that** such apparatus is supported by a supporting structure (14), with adjustable bending means.

3. An apparatus for the extraction of active principles, according to claims 1 or 2, **CHARACTERIZED in that** such transducer system (29), comprises high intensity acoustic transducers.

4. an apparatus for the extraction of active principles, according to claims 1 to 3, **CHARACTERIZED in that** such superficial portion (28) that supports the transducer system (29) has a difference in level with the apparatus structure.

5. an apparatus for the extraction of active principles, according to claim 4, **CHARACTERIZED in that** between such superficial portion and the case (12) a mesh is available (35) that retains the raw material.

6. an apparatus for the extraction of active principles, according to claims 1 to 5, **CHARACTERIZED in that** the acoustic transducer system has excitation means in a self latching ultrasonic generator (32) for the transducers, a signal amplifier (33) and an electromechanical adaptation network (34).

7. an apparatus for the extraction of active principles, according to claims 1 to 6, **CHARACTERIZED in that** it has an engine that inject a rotatory movement to the first helical conveyor screw (13).

8. an apparatus for the extraction of active principles, according to claims 1 to 7, **CHARACTERIZED in that** each vane of such plurality of vanes (17), has slots (18) to increase the contact between the raw material and the solvent extraction means.

9. an apparatus for the extraction of active principles, according to claim 8, **CHARACTERIZED in that** such slots (18) have a radial configuration extending perpendicularly to the rotation axis of the first helical screw. Thus, the possibility of product stagnation is avoided in them.

10. an apparatus for the extraction of active principles, according to claims 1 to 9, **CHARACTERIZED in that** it also comprises temperature control means (20), located around the case (12).

11. an apparatus for the extraction of active principles, according to claim 10, **CHARACTERIZED in that** the heat energy transference means is vapor, water or other similar fluid.

12. an apparatus for the extraction of active principles, according to claims 1 to 11, **CHARACTERIZED in that** it also comprises a second load line (24) and/or a third load line (25) for the solvent extraction load means, which are located in other positions of the surface of the case, between the first means and the load means (23) and the top superficial portion (28).

13. an apparatus for the extraction of active principles, according to claims 1 to 12, **CHARACTERIZED in that** the acoustic field is irradiated from the top superficial portion (28) and from the sides (30) of the case (12).

14. an apparatus for the extraction of active principles, according to claim 15, **CHARACTERIZED in that** such acoustic transducers may have a radiant face in the shape of a disc, stepped horn, plaque or similar.

15. an apparatus for the extraction of active principles, according to claim 16, **CHARACTERIZED in that** such acoustic transducers have a resonance frequency in a range between 15 and 45 kHz.

16. an apparatus for the extraction of active principles, according to claim 15, **CHARACTERIZED in that** such acoustic transducer is located in any section of the apparatus as long as it favors the acoustic cavitation in the extract.

17. an apparatus for the extraction of active principles, according to claims 1 to 16, **CHARACTERIZED in that** the case (12) is bended to an angle of 15°.

18. A method for extracting active principles form natural sources using an extractor on counter-flow assisted by an acoustic transducer system, in an apparatus that allows the applications of an cavitative acoustic field in the flooded area with the material comprised of raw material of the natural product and a solvent extraction means, where such apparatus has at least one inlet of such material, at least one inlet of such solvent extraction means, an outlet of processed residual material and at least one outlet of liquid extract, **CHARACTERIZED in that** it comprises the following stages:
(a) to downsize such material comprised of the raw material of the natural product;
(b) to load such solvent extraction means for at least one inlet of such solvent extraction means in a top end of the apparatus, and load such material for at least one inlet of such material in a bottom end of the apparatus;
(c) to flood with such solvent extraction means and such material until the top portion of the surface (28) located at the bottom end (15) of the apparatus (11), that such acoustic transducer system (29) until its radiant face is submerged;
(d) to apply an acoustic field to the solution formed between the solvent extraction means and the material;
(e) to continue the extraction on counter-flow between at least one inlet of the solvent extraction means of the apparatus and at least one outlet of the processed residual material.
(f) to collect the extracted liquid obtained; and
(g) to collect the exhausted residual material.

19. A method according to claim 18, **CHARACTERIZED in that** to establish the application conditions of the acoustic field, the bottom end of the equipment must be flooded with the extraction liquid and the material.

20. A method according to claims 18 or 19, **CHARACTERIZED in that** such flood level must be constantly kept during the process.

21. A method according to any of the claims 18 to 20, **CHARACTERIZED in that** the exposure time of the raw material to the acoustic field is not higher than the time of extraction on counter-flow.

22. A method according to any of the claims 18 to 21, **CHARACTERIZED in that** it previously include the stage of defining at least the parameters of: granulometry of the product, exposure time to the acoustic field, acoustic power, concentration (solid/liquid) and temperature.

23. A method according to claim 24, **CHARACTERIZED in that** it also includes the definition of at least the parameters: feed rate of the material, bending angle of the apparatus, liquid/insoluble solid relations, feed rate of the solvent extraction means and screw spin speed.

24. A method according to any of the claims 18 to 23, **CHARACTERIZED in that** it also include a partial flood of the rest of the apparatus according to the bending angle.

25. A method according to claim 24 **CHARACTERIZED in that** such angle is selected between the range of 5° to 85°.

26. A method according to claim 25, **CHARACTERIZED in that** such angle is 18°.

27. A method according to any of the claims 18 to 26, **CHARACTERIZED in that** once the extraction on counter-flow is finished, the liquid extract obtained, Rich in active principles, pass through a unload line of the apparatus through a filter (27) installed at the bottom end of the apparatus.

28. A method according to any of the claims 18 to 27, **CHARACTERIZED in that** the exhausted residual material is unloaded through an unload hopper (31) located at the top end of the apparatus.

29. A method according to any of the claims 18 to 28, **CHARACTERIZED in that** the stage (a) also includes reducing the humidity degree of such material through drying means.

30. A method according to any of the claims 18 to 29, **CHARACTERIZED in that** the extraction liquid used is water.

31. A method according to any of the claims 18 to 29, **CHARACTERIZED in that** the extraction liquid used is alcohol, chloroform, methanol, acetone, hydro alcoholic mixtures.

32. A method according to any of the claims 18 to 31, **CHARACTERIZED in that** the solid/liquid relation in the acoustic transducer part must favor the acoustic cavitation.

33. A method according to any of the claims 18 to 32, **CHARACTERIZED in that** the material includes a range of size between 6 and 80-mm.

34. A method according to any of the claims 18 to 33, **CHARACTERIZED in that** in stage (d) the acoustic waves influence frontally the surface of the product.

35. A method according to any of the claims 18 to 34, **CHARACTERIZED in that** it includes a process temperature between approximately 5° C and 80° C.
